# EUROPEAN PATENT APPLICATION

(11) **EP 2 368 524 A2**
(43) Date of publication of application: **28.09.2011**
(21) Application number: 11250365.1
(22) Date of filing: 24.03.2011
(51) Int. Cl.: A61F 2/00

(54) **Hernia patch**

(30) Priority: 25.03.2010 US 317339 P; 28.02.2011 US 36422
(71) Applicant: Tyco Healthcare Group LP, New Haven, CT 06511 (US)
(72) Inventor: Thomas, Jonathan, New Haven, CT 06511 (US); Hotter, Joseph, 69006 Lyon (FR); Stopek, Joshua, Guilford, CT 06437 (US)
(74) Representative: Soames, Candida Jane

(57) **Abstract**

Surgical implants which include a biocompatible substrate and at least one grip member capable of transitioning between a first non-gripping configuration and a second gripping configuration.

## Description

### CROSS-REFERENCE TO RELATED APPLICATIONS

This application claims the benefit of and priority to U.S. Provisional Patent Application No. 61/317,339, filed March 25, 2010, the entire disclosure of which is incorporated by reference herein.

### BACKGROUND

### Technical Field

The present disclosure relates to an implantable hernia repair device, and more particularly, to an implantable hernia patch, which includes a sheet of biocompatible material and grip members made of shape memory polymers.

### Background of Related Art

A hernia is a protrusion of tissue or part of an organ through injured muscle tissue or an injured membrane by which the tissue or organ is normally contained. Hernias are principally repaired by pushing back, or "reducing," the herniated tissue, and then reinforcing the defect in injured muscle tissue, for example by an implant, such as a hernia patch. The implant may be either placed over the defect (anterior repair) or under the defect (posterior repair).

Implants useful for repairing such defects may include a tissue adherent property. The tissue adherent property may be provided, for example, by an adhesive coating or by spiked naps. When used in laparoscopic procedures, an implant with tissue adherent properties may have a tendency to adhere to itself when rolled or crumpled for delivery through a cannula, thereby becoming entangled and difficult to unroll at the site of implantation.

It would desirable to provide an implant for hernia repair, which has tissue adherent properties, yet may be rolled on itself without becoming self-adhered.

### SUMMARY

The present disclosure describes a surgical implant, e.g., hernia patch, including a biocompatible substrate and at least one grip member capable of transitioning between a first non-gripping configuration and a second gripping configuration.

In embodiments, the at least one grip member may be made from a shape memory material. Upon exposure to a stimulus, the grip members transition between two different configurations, a first non-gripping configuration and a second gripping configuration. In embodiments, the biocompatible substrate is a surgical mesh.

Methods of making a surgical implant with tissue adherent properties are also described.

### BRIEF DESCRIPTION OF THE DRAWINGS

Various embodiments of the present disclosure will be discussed in more detail below in conjunction with selected embodiments and the appended drawings wherein:

FIG. 1A shows a perspective view of one embodiment of a hernia patch according to the present disclosure;

FIG. 1B shows a side view of the embodiment shown in FIG. 1A;

FIG. 1C shows a perspective view of another embodiment of the hernia patch according to the present disclosure;

FIG. 1D shows a side view of the embodiment of FIG.1C, grip members in the second configuration;

FIGS. 2A-2B show side views of another embodiment of a hernia patch according to the present disclosure;

FIG. 3A shows a perspective view of yet another embodiment of a hernia patch according to the present disclosure; and

FIG. 3B shows a side view of the embodiment shown in FIG. 3A.

### DETAILED DESCRIPTION

Implants in accordance with the present disclosure include a biocompatible substrate and at least one grip member made of a shape memory polymer which in certain configurations protrudes from a surface of the substrate.

The biocompatible substrate can be in any form that has sufficient strength to serve as a reinforcement for a defect in tissue and the capacity to support one or more gripping members. Suitable forms for the biocompatible substrate include porous or non-porous films, gel sheets or fibrous structures, such as, for example knitted, woven or nonwoven sheets. These various forms may be used alone or in combination with one another. Thus, for example, the substrate may be a composite of one or more of a film, gel and/or fibrous structure. Where temporary support (i.e., stiffness) of the tissue defect is needed, an absorbable material may be used to form all or part of the substrate. Where permanent support of the tissue defect is needed, the biocompatible substrate should be made entirely or in part of a non-absorbable material.

In certain embodiments, the biocompatible substrate is a mesh sheet. The mesh substrates described herein may include porous fabrics made from intertwined filaments. The filaments may extend horizontally and vertically in a manner which produces sections where the filaments cross-over one another creating points of common intersection. The filaments may be monofilaments or multi-filaments and, in embodiments, a plurality of multi-filaments may be combined to form yarns. It is envisioned that the mesh sheet may be configured to any size and/or shape suitable for hernia repair. The filaments may comprise core/sheath constructs.

The mesh substrates described herein may be manufactured out of filaments made from any biocompatible absorbable or non-absorbable material. Some non-limiting examples of absorbable materials used to form the filaments of the mesh substrate include absorbable polymers made from glycolide, glycolic acid, lactide, lactic acid, caprolactone, dioxanone, trimethylene carbonate and dimethyl trimethylene carbonate. Non-absorbable materials used to form the filaments of the mesh substrate may include, but are not intended to be limited to, materials such as nylon, silk, polypropylenes, polyethylene teraphthalate, polybutylene terephthalate, polytetrafluoroethylene, polyurethanes, and polyvinyl chloride. Copolymers (block or random) and mixtures and blends of such biocompatible polymeric or copolymeric materials may also be useful.

The mesh substrate may be formed using any suitable method. For example, the mesh substrate may be woven, non-woven, knitted or braided. In embodiments, the mesh substrate may be knitted on a warp knitting machine, of the tricot or Raschel type, with multiple filaments or yarns and multiple guide-bars. The mesh substrates may be made from any fabric that may be biocompatible and also may be capable of sterilization.

In embodiments, the mesh substrate may be formed by knitting a two-dimensional fabric on a warp knitting machine. Some examples may be found in U.S. Patent No. 7,331,199, the entire content of which is incorporated by reference herein. In other embodiments, the mesh substrate may be formed by knitting a three-dimensional fabric on a knitting machine. Some examples of three-dimensional mesh substrates may be found in U.S. Patent No. 7,021,086; U.S. Patent No. 6,596,002; and U.S. Patent No. 7,331,199 the entire contents of which are incorporated by reference herein.

Implants in accordance with the present disclosure further include at least one grip member protruding from a surface of the biocompatible substrate. The grip member may be made from at least one filament. The grip member may protrude from the surface of the biocompatible substrate in a generally perpendicular direction. In embodiments, a plurality of grip members may be positioned over an entire surface of the biocompatible substrate. In other embodiments, a plurality of grip members may be positioned over only a portion of the surface of the biocompatible substrate. In still other embodiments, the grip members may protrude from more than one surface of the biocompatible substrate.

The grip member(s) may be made of any suitable shape memory material. Suitable shape memory materials include shape memory polymers and shape memory alloys. A variety of shape memory polymers and shape memory alloys, which may be formed into filaments useful as the grip member of the present implant, include, but are not limited to those disclosed herein.

Among the useful shape memory polymers are those that may be conditioned using two separate phases or three separate phases. Two-phase shape memory polymers may be polymers which have both a current form (temporary) and a stored form (permanent). Once the latter has been manufactured by any conventional method, the polymer may be changed into a temporary form by processing the polymer through heating, deformation, and cooling. After cooling, the polymer maintains the temporary shape until the polymer is activated by a predetermined external stimulus. In addition to temperature change, the shape memory polymers can also activated by electric or magnetic fields, light, and/or a change in pH. Upon activation, the polymer shape transitions back to the permanent shape.

In some embodiments, the grip members may consist of polymeric monofilaments manufactured in a curled state. That is to say the permanent shape of the shape memory monofilaments is generally curled. The curled polymeric monofilaments may be heated above the polymer's glass transition temperature (Tg), which allows the polymer to become soft, flexible, and easier to shape. While above the Tg, the curled polymeric monofilaments may be straightened. The straightened polymeric monofilaments may then be cooled below the Tg of the polymer to keep the monofilaments in the straightened, temporary shape. In such embodiments, the straightened polymeric monofilaments may be conditioned to return to the permanent form, i.e., curled, upon exposure to the proper external stimulus, such as a change in temperature above the Tg of the given polymer.

Although specifically recited as a polymeric monofilament, it is envisioned that the grip members may also be formed from a plurality of monofilaments, mutli-filaments and yarns formed by a plurality of multi-filaments. In addition, the shape memory polymers may be combined with any variety of biocompatible materials to form the grip members.

It is envisioned that the polymeric grip members may be conditioned as shape memory polymers in the presence of or the absence of the biocompatible sheet. In embodiments, the grip members may be conditioned prior to being incorporated into the biocompatible sheet. In other embodiments, the grip members may be conditioned after being incorporated into the biocompatible sheet. In still other embodiments, the grip members may be attached to a separate fabric, such as a flap mesh sheet, and the separate fabric, which includes the grip members, may be attached to the biocompatible sheet by any suitable method, including, but not limited to adhesives and stitching.

In some embodiments, the shape memory polymer used to form the grip members may display a Tg ranging from about 20°C to about 40°C. In such embodiments, the grip members will transition from the temporary shape to the permanent shape when exposed to temperatures in this range, such as body temperature.

Some non-limiting examples of suitable polymeric materials that may be used to form the grip members include synthetic materials such as: vinyl polymers including phosphporyl choline, hydroxamates, vinyl furanones, and copolymers thereof; quarternary ammoniums; copolymers of alkylene oxides and lactones; copolymers of alkylene oxides and orthoesters; copolymers of alkylene oxides and hydroxybutyrates; polyurethanes; and polynorbornene. Representative natural biodegradable polymers include: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as albumin, casein, zein, silk, and copolymers and blends thereof, alone or in combination with synthetic polymers.

Synthetically modified natural polymers include cellulose derivatives, such as alkyl celluloses, hydroxyalkyl celluloses, cellulose ethers, cellulose esters, nitrocelluloses, and chitosan. Examples of suitable cellulose derivatives include: methyl cellulose; ethyl cellulose; hydroxypropyl cellulose; hydroxypropyl methyl cellulose; hydroxybutyl methyl cellulose; cellulose acetate; cellulose propionate; cellulose acetate butyrate; cellulose acetate phthalate; carboxymethyl cellulose; cellulose triacetate; and cellulose sulfate sodium salt. These are collectively referred to herein as "celluloses."

Representative synthetic degradable polymers include: polyhydroxy acids prepared from lactone monomers, such as glycolide, lactide, caprolactone, ε-caprolactone, valerolactone, and δ-valerolactone; as well as pluronics; carbonates (e.g., trimethylene carbonate, tetramethylene carbonate, and the like); dioxanones (e.g., 1,4-dioxanone and p-dioxanone); 1,dioxepanones (e.g., 1,4-dioxepan-2-one and 1,5-dioxepan-2-one); and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, and combinations thereof.

In embodiments, combinations of both degradable and non-degradable materials, including those having shape memory characteristics, may be utilized.

In embodiments, the shape memory polymer may be a copolymer of two components with different thermal characteristics, such as oligo (epsilon-caprolactone) dimethacrylates and butyl acrylates, including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002), the entire disclosure of which is incorporated by reference herein.

In other embodiments, blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of suitable shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, Lendlein et al., "Biodegradable, Elastic Shape memory Polymers for Potential Biomedical Applications," Science, Vol. 269 (2002) 1673-1676, and Lendlein et al., "Shape-Memory Polymers," Angew. Chem. Int. Ed., 41 (2002) 2035-2057, the entire disclosures of each of which are incorporated by reference herein.

Table 1 below further illustrates compositions which demonstrate shape memory effects. The block copolymers of each composition are in annealed wire format, the proposed soft and hard segments, and the glass transition temperature (T_{g}), having been measured by differential scanning calorimetry which is equal to T_{Trans}.

**TABLE 1**

| Composition (mol%) | Soft Domain | Hard Domain | T_{g} (T_{Trans}) [°C] |
|---|---|---|---|
| 15% Polydioxanone | Polydioxanone and | Crystalline Polylactide | 54 |
| 85% Poly (L-lactide) | Amorphous Polylactide | | |
| 20% Polydioxanone | Polydioxanone and | Crystalline Polylactide | 45 |
| 80% Poly (L-lactide) | Amorphous Polylactide | | |
| 15% Trimethylene | Trimethylene | Crystalline Polylactide | 54 |
| Carbonate | Carbonate and | | |
| 85% Poly (L-lactide) | Amorphous Polylactide | | |
| 20% Trimethylene | Trimethylene | Crystalline Polylactide | 55 |
| Carbonate | Carbonate and | | |
| 80% Poly (L-lactide) | Amorphous Polylactide | | |

The copolymers in Table 1 may undergo a partial shift when approaching T_{g} and T_{Trans} may be depressed when the materials are in aqueous solution. Since these polymers degrade by water absorption and bulk hydrolysis, water molecules entering the polymer matrices may act as plasticizers, causing the soft segments to soften at lower temperatures than in dry air. Thus, polymers exhibiting T_{Trans} depression in aqueous solution may maintain a temporary shape through temperature excursions in the dry state, such as during shipping and storage, and shape shift to its permanent shape at body temperatures upon implantation.

Thus, in embodiments, the shape memory polymer may include a block copolymer of polydioxanone and polylactide with the polydioxanone present in an amount from about 5 mol% to about 20 mol% of the copolymer, in embodiments from about 15 mol% to about 19 mol% of the copolymer, and the polylactide present in an amount from about 80 mol% to about 95 mol% of the copolymer, in embodiments from about 81 mol% to about 85 mol% of the copolymer. In other embodiments, the shape memory polymer may include a block copolymer of trimethylene carbonate and polylactide, with the trimethylene carbonate present in an amount from about 5 mol% to about 20 mol% of the copolymer, in embodiments from about 15 mol% to about 19 mol% of the copolymer, and the polylactide may be present in an amount from about 80 mol% to about 95 mol% of the copolymer, in embodiments from about 81 mol% to about 85 mol% of the copolymer.

It is envisioned that T_{Trans} may be tailored by changing block segment molar ratios, polymer molecular weight, and time allowed for hard segment formation. In embodiments, T_{Trans} may be tailored by blending various amounts of low molecular weight oligomers of the soft segment domain into the copolymer. Such oligomers may act as plasticizers to cause a downward shift in T_{Trans.}

In some embodiments, the grip members may possess a first, generally straight configuration at a temperature less than about 20°C and may assume a second, generally curled configuration when exposed to a temperature ranging from about 20°C to about 40 °C. In certain embodiments, shape memory polymers exhibit a transition temperature at around 37 °C or body temperature. Although the grip members are disclosed predominantly as transitioning from straight to curled, it well within the scope of the present disclosure to include grip members that may transition to any number of different shapes and dimensions. For example, the grip members may transition from gripping to non-gripping configuration in removal or repositioning of a mesh. The two different configurations between which the grip members can transition may be determined at the time of conditioning the shape memory polymer. In embodiments, the grip members may assume any shape suitable for providing tissue adherent properties to the patch. For instance, the grip members may transition from a temporary straight configuration to a permanent L-shaped configuration. In the most general sense, upon exposure to a stimulus, the grip members transition between two different configurations, a first, non-gripping configuration and a second, gripping configuration.

In other embodiments, the shape memory polymer may be a light activated shape memory polymer. Light activated shape memory polymers (LASMP) may use processes of photo-crosslinking and photo-cleaving to change the glass transition temperature (Tg). Photo-crosslinking may be achieved by using one wavelength of light, while a second wavelength of light reversibly cleaves the photo-crosslinked bonds. The effect achieved may be that the material may be reversibly switched between an elastomer and a rigid polymer. Light may not change the temperature, but may change the cross-linking density within the material. For example, polymers containing cinnamic groups may be fixed into predetermined shapes by UV light illumination (> 260 nm) and then recover their original shape when exposed to UV light of a different wavelength (< 260 nm). Non-limiting examples of photoresponsive switches include cinnamic acid and cinnamylidene acetic acid.

Suitable shape memory alloys capable of being spun into filaments that can be used to form the grip members include, but are not limited to, nitinol (NiTi), CuZnAl, CuAlNi and FeNiAl. Methods for forming fibers from shape memory alloys are within the purview of those skilled in the art.

In addition to providing tissue support and tissue ingrowth, the patches may further be used for delivery of a bioactive agent. Thus, in some embodiments, at least one bioactive agent may be combined with the sheet, the grip member(s) or both. The agents may be physically admixed with the biocompatible materials used to form the sheet or grip members, coated onto the sheet or grip members or tethered to the biocompatible materials used to form the sheet or grip members through any variety of chemical bonds. In these embodiments, the present implant may also serve as a vehicle for delivery of the bioactive agent.

The hydrogel may be coated with or include additional bioactive agents. The term "bioactive agent," as used herein, is used in its broadest sense and includes any substance or mixture of substances that have clinical use. Consequently, bioactive agents may or may not have pharmacological activity per se, e.g., a dye. Alternatively a bioactive agent could be any agent, which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the hydrogel in any suitable form of matter, e.g., films, powders, liquids, gels and the like.

As noted above, in embodiments that include a multi-arm PEG or PEG star, the bioactive agent may be incorporated into the core of the PEG, the arms of the PEG, or combinations thereof. In embodiments, the bioactive agent may be attached to a reactive group in the PEG chain. The bioactive agent may be bound covalently, non-covalently, i.e., electrostatically, through a thiol-mediated or peptide-mediated bond, or using biotin-adivin chemistries and the like.

Examples of classes of bioactive agents, which may be utilized in accordance with the present disclosure include, for example, anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Anti-adhesive agents can be used to prevent adhesions from forming between the hydrogel and the surrounding tissues opposite the target tissue. In addition, anti-adhesive agents may be used to prevent adhesions from forming between the coated implantable medical device and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents which may be included as a bioactive agent include, for example, triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent.

Other bioactive agents, which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents, which may be included in the hydrogel include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

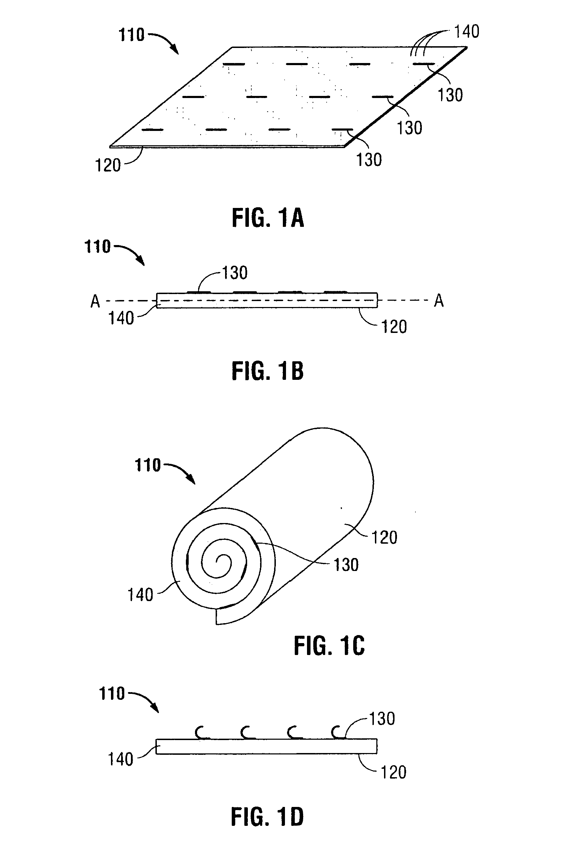

Turning now to the illustrative embodiment of FIGS. 1A through 1D, surgical implant 110 includes biocompatible substrate 120 and a plurality of grip members 130. Surgical implant 110 may be generally planar and includes a plurality of pores 140. Biocompatible substrate 120 is shown as a mesh fabric formed from knitted filaments. In Figure 1B, grip members 130 are shown positioned along a top surface of substrate 120 in a non-gripping position. In a non-gripping position, grip members 130 are in a generally straight manner positioned parallel to the longitudinal axis (A) of substrate 120. Because the grip members run generally parallel to the axis of substrate 120, grip members 130 are unable to penetrate into the surrounding tissue upon implantation or penetrate into other portions of substrate 120. In the non-gripping position, grip-members 130 are unable to attach to other portions of substrate 120 and are also unable to attach to the surrounding tissue upon implantation. Although shown as generally parallel to the longitudinal axis (A) of substrate 120, grip members 130 do not have to be generally parallel. In embodiments, the grip members may be in any position relative to the longitudinal axis of the substrate which may be suitable for preventing the grip members from attaching to the tissue and/or other portions of the substrate.

As shown in Figure 1C, implant 110 including grip members 130 in a non-gripping configuration, may be rolled into a tubular configuration wherein several different portions of substrate 120 overlap one another, without grip members 130 becoming entangled within pores 140 of substrate 120. During a laparoscopic procedure, implant 110 may be passed through a cannula (not shown) in the rolled configuration prior to implantation and may be unrolled at the site of implantation because grip-members 130 do not attach to substrate 120 in the non-gripping configuration.

After implantation, implant 110 may be warmed by the body to a temperature of above 20°C. At which time grip-members 130, made of a shape memory material which has been conditioned to transition above a temperature of about 20°C, will transition from a non-gripping configuration to a gripping configuration (see Figure 1D). In the gripping configuration of Figure 1D, grip members 130 have transitioned from a generally flat and straight configuration to a generally curled, gripping configuration. It should be understood that the shape memory materials may be conditioned or manufactured to transition at specific temperatures, such as, for example body temperature (about 37°C).

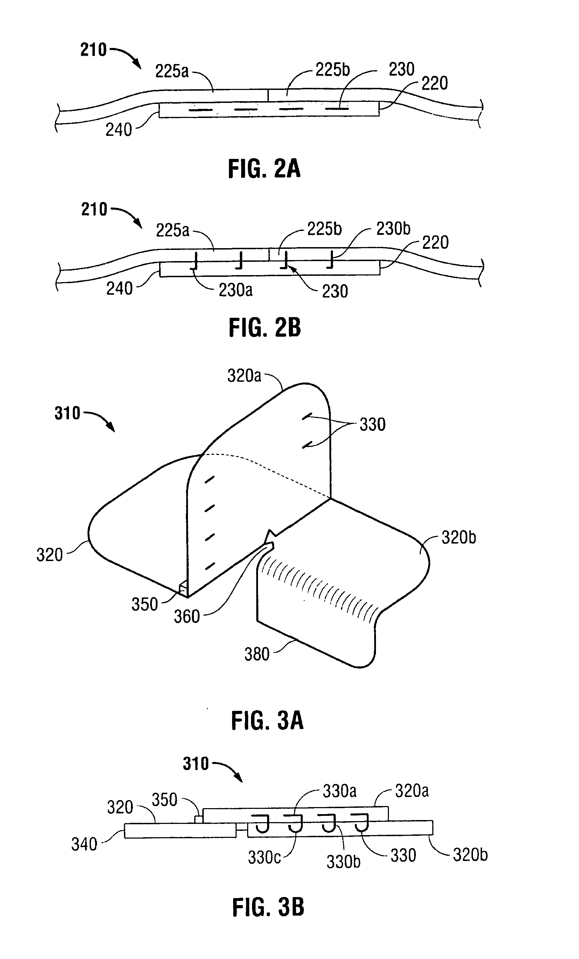

In FIGS. 2A and 2B, surgical implant 210 is shown at the site of implantation in a substantially planar position adjacent first and second approximated portions of wound tissue 225a, 225b. Grip members 230 are in a non-gripping position prior to exposure to a predetermined stimulus. For example, the substrate may be kept at a temperature of about 20°C or less.

As shown in FIG. 2B, the opening in the injured tissue may be closed by approximating a first side 225a of the injured tissue with a second side 225b of the injured tissue. Grip members 230 will transition from the non-gripping configuration to a gripping configuration following exposure to the stimulus, such as an increase in temperature to between about 20°C and about 40°C. As depicted in Fig. 2B, grip members 230 may form a generally L-shaped configuration wherein base member 230a of grip member 230 is positioned within a portion of substrate 220 and arm member 230b of grip member 230 extends generally perpendicular from the longitudinal axis of substrate 220 to attach substrate 220 to tissue 225a, 225b.

In other embodiments, grip members 330 may be utilized to securely anchor a first portion 320a of substrate 320 to a second portion 320b of substrate 320. In FIGS. 3A and 3B, implant 310 includes a mesh substrate 320 made from filaments which are intertwined in manner which creates a plurality of pores 340. Mesh substrate 320 also includes slit 360, which extends generally from the center of mesh substrate 320 to an outer edge 380 of mesh substrate 320. First portion 320a of substrate 320 may be attached to substrate 320 via seam 350, and may be intended to overlap second portion 320b of substrate 320. First portion 320a includes a plurality of grip members 330. Grip members 330 are conditioned to transition from a non-gripping configuration (FIG.3A) to a gripping configuration (FIG.3B) upon exposure to a predetermined stimulus as described herein.

In FIG. 3B, implant 310 is shown in a side view with first portion 320a positioned on second portion 320b of substrate 320 wherein grip members 330 have transitioned into a gripping configuration. Grip members 330 have transitioned from a generally straight configuration parallel to the longitudinal axis of substrate 320 to a generally J-shaped configuration, as depicted in FIG. 3B. Generally J-shaped grip members 330 include base member 330a positioned within substrate 320 connected to arm member 330b which extends generally perpendicular from the longitudinal axis of substrate 320. Arm member 330b is designed to penetrate into pores 340 of substrate 320. The distal most portion of arm member 330b includes a curled end 330c. Curled end 330c is designed to engage at least one of the filaments of substrate 320 to hold first portion 320a adjacent to second portion 320b of substrate 320.

Substrates containing shape memory grip members may be combined with other substrates which may or may not include grip members. For example, a mesh having grip members may be used in combination with another mesh not having grip members.

Methods of making a hernia patch with tissue adherent properties are disclosed. The methods include conditioning a grip member that extends from a biocompatible sheet to assume a first, non-gripping configuration, and upon exposure to a stimulus assume a second, gripping configuration.

It will be understood that various modifications may be made to the embodiments disclosed herein. For example, in embodiments the implant may include additional layers of materials, such as an antimicrobial coating layer, or a film for preventing adhesion formation on a portion of the patch. Thus, those skilled in the art will envision other modifications within the scope and spirit of the claims.

The invention may be described by reference to the following numbered paragraphs:-
1. A surgical implant comprising:
   a biocompatible substrate; and
   at least one grip member capable of transitioning between a first non-gripping configuration and a second gripping configuration.
2. The surgical implant of paragraph 1 wherein the grip member is interwoven with the biocompatible substrate.
3. The surgical implant of paragraph 1 wherein the grip member comprises a shape memory material.
4. The surgical implant of paragraph 3 wherein the shape memory material transitions from the first configuration to the second configuration between about 20° C and about 40° C.
5. The surgical implant of paragraph 3 wherein the shape memory material is selected on the group consisting of polymers and metal alloys.
6. The surgical implant of paragraph 5 wherein the shape memory polymer is selected from the group consisting of polyurethanes, poly(styrene-butadiene) block copolymers, polynorbornenes, polycaprolactones, polydioxanones, polylactic acids, oligo (p-dioxanone) diols, oligo (epsilon caprolactone) diols, polytrimethylene carbonates, and combinations thereof.
7. The surgical implant of paragraph 5 wherein the shape memory polymer comprises copolymers selected from the group consisting of poly(epsilon-caprolactone) dimethacrylate-poly (n-butyl acrylate), poly(epsilon caprolactone) diol-poly(p-dioxanone) diol, and combinations thereof.
8. The surgical implant of paragraph 3 wherein the shape memory material comprises a light-activated shape memory polymer.
9. The surgical implant of paragraph 3 wherein the shape memory material comprises a heat-activated shape memory material.
10. The surgical implant of paragraph 3 wherein the shape memory material comprises an electrically activated shape memory material.
11. The surgical implant of paragraph 5 wherein the shape memory metal alloy comprises an alloy selected from the group consisting of copper-zinc-aluminum-nickel alloys, copper-aluminum-nickel alloys, zinc-copper-gold-iron alloys, and nickel-titanium (NiTi) alloys.
12. The surgical implant of paragraph 1 wherein the biocompatible substrate is selected from the group consisting of a foam, film, tissue scaffold, pledget, buttress, mesh and combinations thereof.
13. The surgical implant of paragraph 1 wherein the biocompatible substrate comprises a soft tissue repair device.
14. The surgical implant of paragraph 1 wherein the biocompatible substrate comprises a surgical mesh.
15. The surgical implant of paragraph 1 further comprising a bioactive agent.
16. The surgical implant of paragraph 1 wherein the first non-gripping configuration of the grip member is positioned substantially parallel to a longitudinal axis of the biocompatible substrate.
17. The surgical implant of paragraph 1 wherein the second gripping configuration of the grip member extends substantially perpendicular from a longitudinal axis of the biocompatible substrate.
18. The surgical implant of paragraph 1 wherein the second gripping configuration comprises a generally L-shaped grip member.
19. The surgical implant of paragraph 1 wherein the second gripping configuration comprises a generally J-shaped grip member.
20. A method of making a surgical implant, the method comprising:
   conditioning at least one grip member to transition between a first, non-gripping configuration and a second, gripping configuration; and
   incorporating the at least one grip member into a biocompatible substrate.
21. The method of paragraph 20 wherein conditioning the at least one grip member comprises heating the grip member, deforming the grip member to a non-gripping configuration and cooling the grip member.
22. The method of paragraph 20 wherein incorporating the at least one grip member into a biocompatible substrate is selected from the group consisting of knitting, weaving, braiding, and combinations thereof.
23. The method of paragraph 20 wherein the at least one grip member comprises a shape memory material.
24. The method of paragraph 23 wherein the shape memory material has a temperature transition between about 20° C and about 40° C.
25. The method of paragraph 23 wherein the shape memory material is selected from the group consisting of shape memory polymers and shape memory alloys.
26. The method of paragraph 20 wherein the biocompatible substrate is selected from the group consisting of a foam, film, tissue scaffold, pledget, buttress, mesh and combinations thereof.
27. The method of paragraph 20 wherein the biocompatible substrate comprises a surgical mesh.

## Claims

1. A surgical implant comprising:
a biocompatible substrate; and
at least one grip member capable of transitioning between a first non-gripping configuration and a second gripping configuration.

2. The surgical implant of claim 1 wherein the grip member is interwoven with the biocompatible substrate.

3. The surgical implant of claim 1 or claim 2 wherein the grip member comprises a shape memory material; preferably wherein the shape memory material transitions from the first configuration to the second configuration between about 20° C and about 40° C.

4. The surgical implant of claim 3 wherein the shape memory material is selected on the group consisting of polymers and metal alloys.

5. The surgical implant of claim 4 wherein the shape memory polymer is selected from the group consisting of polyurethanes, poly(styrene-butadiene) block copolymers, polynorbornenes, polycaprolactones, polydioxanones, polylactic acids, oligo (p-dioxanone) diols, oligo (epsilon caprolactone) diols, polytrimethylene carbonates, and combinations thereof; preferably wherein the shape memory polymer comprises copolymers selected from the group consisting of poly(epsilon-caprolactone) dimethacrylate-poly (n-butyl acrylate), poly(epsilon caprolactone) diol-poly(p-dioxanone) diol, and combinations thereof.

6. The surgical implant of claim 3 wherein the shape memory material comprises a light-activated shape memory polymer; and/or wherein the shape memory material comprises a heat-activated shape memory material; and/or wherein the shape memory material comprises an electrically activated shape memory material.

7. The surgical implant of claim 6 wherein the shape memory metal alloy comprises an alloy selected from the group consisting of copper-zinc-aluminum-nickel alloys, copper-aluminum-nickel alloys, zinc-copper-gold-iron alloys, and nickel-titanium (NiTi) alloys.

8. The surgical implant of any preceding claim wherein the biocompatible substrate is selected from the group consisting of a foam, film, tissue scaffold, pledget, buttress, mesh and combinations thereof; preferably wherein the biocompatible substrate comprises a soft tissue repair device or a surgical mesh.

9. The surgical implant of any preceding claim further comprising a bioactive agent.

10. The surgical implant of any preceding claim wherein the first non-gripping configuration of the grip member is positioned substantially parallel to a longitudinal axis of the biocompatible substrate.

11. The surgical implant of any preceding claim wherein the second gripping configuration of the grip member extends substantially perpendicular from a longitudinal axis of the biocompatible substrate.

12. The surgical implant of any of claims 1 to 10 wherein the second gripping configuration comprises a generally L-shaped grip member; or wherein the second gripping configuration comprises a generally J-shaped grip member.

13. A method of making a surgical implant, the method comprising:
conditioning at least one grip member to transition between a first, non-gripping configuration and a second, gripping configuration; and
incorporating the at least one grip member into a biocompatible substrate.

14. The method of claim 13 wherein conditioning the at least one grip member comprises heating the grip member, deforming the grip member to a non-gripping configuration and cooling the grip member.

15. The method of claim 13 or claim 14 wherein incorporating the at least one grip member into a biocompatible substrate is selected from the group consisting of knitting, weaving, braiding, and combinations thereof.

16. The method of any of claims 13 to 15 wherein the at least one grip member comprises a shape memory material.

17. The method of claim 16 wherein the shape memory material has a temperature transition between about 20° C and about 40° C.

18. The method of claim 16 or claim 17 wherein the shape memory material is selected from the group consisting of shape memory polymers and shape memory alloys.

19. The method of any of claims 13 to 18, wherein the biocompatible substrate is selected from the group consisting of a foam, film, tissue scaffold, pledget, buttress, mesh and combinations thereof, preferably wherein the biocompatible substrate comprises a surgical mesh.
